# EUROPEAN PATENT SPECIFICATION

(11) **EP 4 185 203 B1**
(45) Date of publication and mention of the grant of the patent: **29.07.2026**
(21) Application number: 21751850.5
(22) Date of filing: 23.07.2021
(51) Int. Cl.: A61B 5/16, G10L 25/63, A61B 5/00, G16H 50/20, G16H 20/70

(54) **CONTEXT AWARE ASSESSMENT**
KONTEXTBEWUSSTE BEURTEILUNG
ÉVALUATION SENSIBLE AU CONTEXTE

(30) Priority: 23.07.2020 GB 202011453
(43) Date of publication of application: 31.05.2023
(73) Proprietor: BlueSkeye AI Ltd., Nottingham NG7 2TU (GB)
(72) Inventor: VALSTAR, Michel, Nottingham Nottinghamshire NG7 2TU (GB)
(74) Representative: Barker Brettell LLP
(86) International application number: PCT/GB2021/051904
(87) International publication number: WO 2022/018453

(56) References cited:
- US-A1- 2017 238 859
- US-A1- 2019 385 711

## Description

### FIELD OF THE INVENTION

The present invention relates to a computer implemented cognitive state estimation method and associated system.

### BACKGROUND

Behaviour data may comprise data relating to a person's body pose, gestures, facial expressions, head actions, gaze behaviour, tone of voice, speech rate, timbre, or a combination thereof.

A trained medical professional, such as a psychologist or a psychiatrist, or an automated machine-learned system, may be able to determine or estimate an emotional or internal cognitive state (e.g. the affective dimension 'valence') of the person from the person's expressive visual behaviour or from a video (with or without audio) or image comprising the behaviour data. A person's mood can be determined based on the presence or absence of laughter, for example.

To automate the process of predicting the emotional or internal cognitive state, statistical machine learning methods may be employed.

The person's expressive visual behaviour may be affected by factors unknown to the assessor (i.e. psychologist or a psychiatrist, or an automated machine-learned system). Reviewing a person's behaviour remotely may result in nuance being missed, which may be apparent to a trained medical professional in the presence of the person, for example.

In behaviomedics, automatic analysis and synthesis of affective and social signals are used to aid objective diagnosis, monitoring, and treatment of medical conditions that alter a subjects affective and socially expressive behaviour.

EP2660745A2 discloses determining a user's mental health by tracking a user's digital activities (for example, using social media) by identifying a baseline pattern - the pattern may include the time of day when the usage occurs, frequency of usage, duration, content (for example, keywords) and location of the user for example. Monitoring is unknown to the user and without requiring his active participation. US20110066036A1 discloses addressing mental health conditions using a mobile processing device - a software application is configured to employ peripheral devices associated with the mobile processing device to provide a user with a self-treating option for addressing mental conditions. When activated, the application provides one more of an interactive diagnosis routine configured to assist in discovering a mental state or condition of the user, and an interactive instructional routine configured to remedy a current mental state.

US 2019/0385711 A1 discloses a computer-implemented system for assessing a user's mental health state by collecting behavioural and contextual data from recordings and other sources and analysing the data using machine-learning models to generate an assessment output.

Although progress has been made, behaviomedics remains a challenging field and there is room for improvement.

### SUMMARY

According to a first aspect of the present disclosure, there is provided a computer implemented cognitive state estimation method as set out in claim 1.

Behaviour primitives are atomic elements of expressive behaviour that can be objectively coded. Behaviour primitives may be understood by human observers. Key examples are facial muscle actions, such as Facial Action Coding System (FACS) Action Units (AUs) - FACs describes all visually identifiable facial movements into individual components (AUs) - individual spoken words, head nods, or the presence or absence of laughter, sighs, hiccups, and other non-verbal signals. Behaviour primitives are not necessarily facial movements - behaviour primitives may be any visible or audible response the user may make to a task or question, or any visible or audible reactions while a task is being set or a question is being asked.

Behaviour primitives may also comprise pose and posture, or other muscle movements.

A behaviour descriptor is a numerical descriptor of the behaviour primitive. For example, the behaviour descriptor may be a number indicating the intensity or frequency that the user demonstrates the behaviour primitive.

A context descriptor is a numerical descriptor of the context primitive. Context may be a non-behavioural parameter that may affect the user and/or the recording of the user. For example, context may include the location, time or date of the recording, the weather at the time of the recording, or may include one or more features of a task or question presented to the user, for example.

The data derived from the behaviour map and the context map may comprise or consist of the behaviour map and the context map.

The estimate of cognitive state may comprise a depression score.

Estimating a cognitive state comprises combining the behaviour map and the context map to create a joint behaviour and context map.

Creating a joint behaviour map may connect a behaviour primitive with a relevant context primitive - for example, context that applied at the time the user displayed the behaviour.

Combining the behaviour map and the context map comprises multiplying the behaviour map and the context map.

Estimating the cognitive state of the user may comprise using data derived from the behaviour map, context map and the joint behaviour and context map.

The data derived from the behaviour map and the context map may be the joint behaviour and context map or may be derived from the joint behaviour and context map.

Producing the behaviour map comprises performing a Fourier transform on the at least one behaviour primitive; and producing the context map comprises performing a Fourier transform on the at least one context primitive.

The behaviour map may include multiple behaviour primitives and the context map may include multiple context primitives. The method may comprise determining if the behaviour map or the context map has fewer primitives than the other, and inserting additional all-zero primitives to the map having fewer primitives where primitives are missing, to create a behaviour map and a context map of equal size.

The data may comprise stacked 2D data comprising a channel for each of the maps.

The method may include stacking the maps to create stacked 2D data (e.g. so as to produce a data cuboid with a layer per map). The method may include stacking the behaviour map, the context map and the joint behaviour and context map. The stacked map may provide an input for analysis by a machine learner.

**In** one example, the method may include extracting multiple behaviour primitives from the recording, assigning each behaviour primitive a single fixed-length descriptor per unit time, performing a Fourier transform and producing a behaviour map. The method may include providing a context primitive and/or extracting a context primitive from the recording, performing a Fourier transform and producing a context map, and - multiplying the behaviour map and the context map to create a joint behaviour and context map.

Estimating the behaviour of the user may comprise using a convolutional neural network. Using a convolutional neural network may comprise providing the joint behaviour and context map, the behaviour map and/or the context map to the convolutional neural network.

According to a second aspect, there is provided a computer implemented cognitive state estimation method, comprising:
receiving a behaviour map, the behaviour map produced from a plurality of behaviour primitives extracted from a recording of a user;
estimating a behaviour from data comprising the behaviour map, wherein the estimating comprises using at least one neural process on the data.

**In** the method of the first aspect estimating a behaviour of the user may comprise using a neural process. A neural process is a neural latent variable model, which combines benefits from neural networks and Gaussian processing, described in more detail below.

The optional features below are applicable to either the first aspect or the second aspect.

Estimating a behaviour may comprise using a convolutional neural network and the neural process may receive an output from the convolutional neural network.

The neural network may be a deep neural network or another neural network suitable for identifying patterns in data. The method may include using a neural process on an output of the neural network.

Using a neural process may comprise using a global latent variable and a decoder to estimate the behaviour from the data.

The global latent variable may have been determined by:
encoding conditions by receiving a plurality of condition descriptor pairs, each condition descriptor pair comprising a true behaviour of a user and data derived from a behaviour map and/or context map for that user;
aggregating the encoded conditions.

Aggregating the encoded conditions may include averaging encoded conditions for each condition descriptor pair.

The method may include flattening the stacked maps to a 1D vector; using the 1D vector directly as input for the neural process; and using the neural process to generate a cognitive state estimation.

The recording of the user may have been obtained by an interaction with a virtual agent. The virtual agent may be an avatar displayed to the user on a display screen, for example. The virtual agent may present the user with a task and/or a question. The recording may include the user's response.

The recording may comprise an audio recording and/or a video recording of the user.

The method may include interacting with the user and obtaining the recording of the user.

The at least one behaviour primitive may comprise a face behaviour primitive. A face behaviour primitive is a behaviour primitive including movement of the facial muscles, for example raising an eyebrow or raising or dropping the corner of the lips.

The at least one behaviour primitive may comprise a voice behaviour primitive. A voice behaviour primitive is a behaviour primitive derivable from the user's voice - for example, from the recording of the person's voice. The voice behaviour primitive may be the pitch of the voice or the rate of speech of the user, for example.

A first behaviour primitive may be a face behaviour primitive and a second behaviour primitive may be a voice behaviour primitive, for example.

Another aspect may provide a system for estimating a cognitive state of a user, the system comprising:
a processor configured to:
receive a recording of a user and at least one context primitive, each context primitive comprising a time series of context descriptors;
extract at least one behaviour primitive from the recording, each behaviour primitive comprising a time series of behaviour descriptors;
produce a behaviour map from the at least one behaviour primitive;
produce a context map from the at least one context descriptor; and
estimate a cognitive state of the user using data derived from the behaviour map and the context map.

The system may be configured to perform the method of the first aspect.

The system may comprise a mobile computing device configured to generate the virtual agent that interacts with the user. The mobile computing device may be configured to produce the recording of the user during interactions with the virtual agent.

The virtual agent may be configured to assign a task to the user and/or ask the user at least one question. The user's response to the task and/or question may be recorded and the recording may be processed as described with reference to the first aspect (including any optional features thereof). The virtual agent may be configured to ask a series of questions and the recording may include all of the user's answers.

The mobile computing device may be a smartphone, tablet, laptop computer or desktop computer. The mobile computing device may include a display screen to display the avatar to the user and/or to interact with the user. The mobile computing device may include a recording device to create the recording.

The virtual agent may be configured to deliver feedback to the user and the mobile computing device may be configured to monitor digitally delivered feedback in real-time. The feedback may be a message instructing the user to book an appointment with a specialist, for example. The feedback may include contact information of a professional, to allow the user to seek advice, for example. The feedback may be based on the estimated cognitive state - for example, the depression score.

Another aspect may provide a non-transitory computer readable medium having stored thereon software instructions that, when executed by a processor, cause the processor to perform the method.

### BRIEF DESCRIPTION OF THE DRAWINGS

Embodiments will now be described, purely by way of example, with reference to the accompanying drawings, in which:
FIG. 1 illustrates a computer implemented cognitive state estimation method according to an embodiment;
FIG.2 illustrates an example cognitive state estimation system;
FIG.3 illustrates a table showing extracted behaviour primitives given a single fixed-length descriptor per unit time of an assessment;
FIG.4a illustrates an example amplitude component of behaviour primitive frequencies computed with Fourier transform;
FIG.4b illustrates an example phase component of behaviour primitive frequencies computed with Fourier transform;
FIG.5 illustrates a table showing context primitives given a single fixed-length descriptor per unit time of an assessment;
FIG.6 illustrates an example method to create a joint behaviour and context map;
FIG.7 illustrates an example method to create a stack of maps;
FIG.8 illustrates an example cognitive state estimation method;
FIG. 9 illustrates evaluation results of context features for depression score prediction on the AVEC 2014 database;
FIG.10 illustrates RMSE error score on AVEC 2014 Freeform task;
FIG.11 illustrates RMSE error score on AVEC 2014 Northwind task;
FIG.12 illustrates PCC correlation score on AVEC 2014 Freeform task;
FIG.13 illustrates PCC correlation score on AVEC 2014 Northwind task;
FIG.14 illustrates ICC correlation score on AVEC 2014 Freeform task;
FIG.15 illustrates ICC correlation score on AVEC 2014 Northwind task;
FIG.16 illustrates an exemplary diagram of using CNNs in combination with NPs to make behaviomedical estimates;
FIG.17 illustrates an exemplary diagram of the neural process implementation;
FIG.18 illustrates evaluation results of Neural Processes against Neural Networks for depression score prediction on the AVEC 2014 database;
FIG.19 illustrates RMSE error score on AVEC 2014 Freeform task;
FIG.20 illustrates RMSE error score on AVEC 2014 Northwind task;
FIG.21 illustrates PCC correlation score on AVEC 2014 Freeform task;
FIG.22 illustrates PCC correlation score on AVEC 2014 Northwind task;
FIG.23 illustrates ICC correlation score on AVEC 2014 Freeform task;
FIG.24 illustrates ICC correlation score on AVEC 2014 Northwind task.

### DETAILED DESCRIPTION

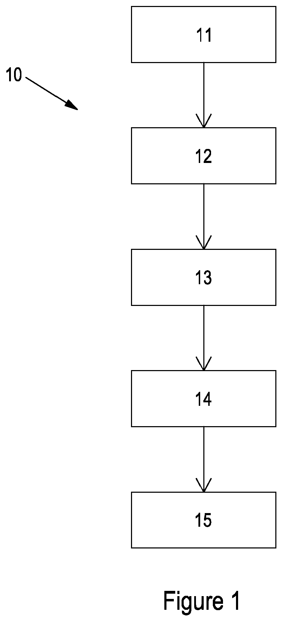

Figure 1 shows a computer implemented method 10 for estimating a cognitive state of a user according to an embodiment, comprising steps 11 to 15. The cognitive state may comprise a mental health state which may be useful for determining a mental health classification.

It may be apparent from facial movements or other physical cues that a person may suffer from or is showing signs of suffering from a medical condition. Example medical conditions include, but are not limited to, Major Depression Disorder, Generalised Anxiety Disorder, ADHD, ASD and pain. A person may demonstrate that they are in physical pain by flinching or holding their head to indicate a headache, for example. Analysing a person's behaviour when faced with a task or questions may allow the person to be diagnosed with a medical condition, assisting in identifying that a person does not have a medical condition, or may be useful in predicting the behaviour of a person with a medical condition, for example.

The medical condition may be depression, the identification of which may be assisted with a depression score. Examples of existing depression scores include: a Global Assessment of Functioning (GAF) score, a Beck Depression Inventory (BDI) score, a Beck Hopelessness Scale score, a Centre for Epidemiological Studies - Depression Scale (CES-D) score, a Center for Epidemiological Studies Depression Scale for Children (CES-DC) score, a Edinburgh Postnatal Depression Scale (EPDS) score, a Geriatric Depression Scale (GDS) score, a Hamilton Rating Scale for Depression (HAM-D) score, a Hospital Anxiety and Depression Scale score, a Kutcher Adolescent Depression Scale (KADS) score, a Major Depression Inventory (MDI) score, a Montgomery-Åsberg Depression Rating Scale (MADRS) score, a PHQ-9 score, a Mood and Feelings Questionnaire (MFQ) score, a Weinberg Screen Affective Scale (WSAS) score, a Zung Self-Rating Depression Scale equivalent score, and a combination thereof. Embodiments of the present method may enable one or more of these scores to be determined (but is not restricted to these).

At step 11, a recording of a user is received. The recording may be obtained by recording the user while being interviewed, performing a task, and/or responding to questions. In certain embodiments, a virtual agent interacts with the user being assessed. The virtual agent may also be referred to as an avatar, and may be presented on a display (e.g. of a tablet or smartphone) and/or embodied as a physical robot (which may comprise a display through which the robot can emote). The virtual agent interviews the user, and/or guides the user through one or more tasks (which may be specifically designed to elicit behaviour primitives in temporal activation patterns that are strong indicators of one or more behaviomedical conditions). The recording of the user preferably comprises both visual information and audio information.

Step 11 further comprises receiving one or more context primitives, each context primitive comprising a time series of context descriptors. In the present application, context refers to features of the environment (including the avatar) and/or user that may elicit or affect the behaviours that can be observed in the recording, and may include: the content of questions that are put to the user (e.g. verbal content, emotional content); the avatar's audio visual properties (e.g. facial expression, tone of voice, speech rate, posture) ambient conditions (current time, weather, geographical location, type and volume of background music, ambient noise levels etc); a task state (e.g. a current score, time taken on a particular task, time taken to achieve a goal). At least some content descriptors may be possible to extract from the recording (e.g. the gender of the user may be recognised from video recording, verbal content of questions from the avatar from audio processing).

At step 12, behaviour primitives are extracted from the recording (each behaviour primitive comprising a time series of behaviour descriptors), for example using a convolutional neural network.

Behavioural primitives may be observed while a person performs a particular task, or is asked questions that elicit a response, for example. How the person acts during the task and/or when considering the question and/or responding may be analysed by identifying that the person has exhibited one or more behaviour primitives. The frequency with which the one or more behaviour primitives are exhibited, or the point in time that the one or more behaviour primitives are exhibited, may be identified and may be used in analysing the person. Only one behaviour primitive may be identified and analysed; however, identifying and analysing multiple behaviour primitives may provide a more useful insight into the person's emotional or physical state during the assessment.

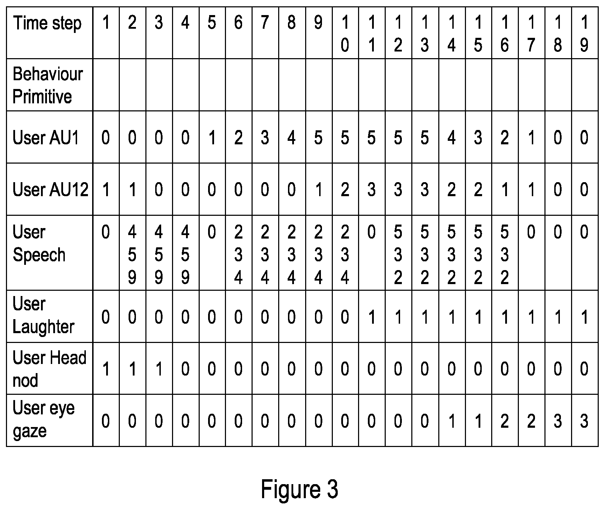

A behaviour primitive may comprise an action unit (AU), for example a Facial Action Coding System AU. Example behaviour primitives with example identifiers are lip corner pulling (AU12), cheeks raise (AU6), eyebrow lowering (AU04). These are AUs from FACs; however any alphanumeric identifier may be chosen to classify a behaviour primitive/action unit. In the example of Figure 3, behavioural primitives are identified by either 'AUX' where X is a number, or by a word. Figure 3 shows a table including example behaviour primitives - User AU1, User AU12, User Speech, User Laughter, User Head nod and User Eye gaze.

The avatar may interact with the user at a time during the assessment (e.g. to ask a question), and the user may respond (e.g. answer the question) at a subsequent time. The response may be analysed using a computer implemented process (e.g. using a convolutional neural network) for the presence of one or more behaviour primitives. Identified behaviour primitives may be set out in a time series, showing what the behaviour primitives are for each unit of time in the assessment (e.g. at a fixed sample rate). A behaviour primitive may be assigned a single fixed-length descriptor per unit time.

The times at which the behaviour primitive(s) occur - which may be never (i.e. the behaviour primitive(s) are not exhibited) - may indicate a pattern. The user's response to questions and/or tasks, and any pattern that the person exhibits, may be used to determine a score that may assist in diagnosis of a medical condition.

Any suitable machine learning algorithm may be used to extract behaviour primitives from the recording. For example a CNN may be used to extract the behaviour primitives.

At step 13, behaviour primitives are combined to form a behaviour map. For example, the behaviour map may comprise a 2D array of data, with each row corresponding with a time series of a specific behaviour primitive. The behaviour map is produced by Fourier transforming each behaviour primitive, and assembling the amplitudes and/or phases into a 2D map. Analysis of spectral content may provide additional insight over time based analysis, and has a further advantage of being able to produce a behaviour map that is of a fixed size from recordings with variable duration. This may simplify the methodology for processing the behaviour map in subsequent steps.

At step 14, a context map is formed, in the same way that the behaviour map was formed in step 13 (e.g. by combining context primitives, and by combining the results from Fourier transforming the context primitives). The context map has the same dimensions as the behaviour map, in accordance with the invention.

At step 15, a cognitive state of the user is determined using data derived from the behaviour map and the context map. The use of context information with the behaviour information may provide enhanced accuracy in estimating cognitive state (over approaches that use behaviour information alone). The cognitive state may be determined using machine learning, for example using a convolutional neural network (CNN).

The cognitive map and behaviour map are combined by multiplication of the behaviour map with the context map to produce a joint behaviour and context map. The joint behaviour and context map may be provided to a machine learning algorithm (e.g. a CNN or other machine learning algorithm), along with the behaviour map (and optionally the context map).

A CNN is a machine learning algorithm, and a type of Artificial Neural Network (ANN), in which convolution operations are used in at least some layers. CNNs learn based on examples - the network receives inputs and known results and forms probability-weighted associations between them. Once the network has received enough examples, the network can begin to predict results from inputs. The network may be provided with a feedback loop, allowing the network to improve its predictions based on its own earlier predictions. For example, CNNs can identify a pattern (for example, in an image) and make a decision based on the pattern, and, in the method, statistical machine learning may be employed to find a predictive function based on behaviour and context from a user recording.

Artificial Neural networks may be aggregated into layers. A first layer may be configured as an input layer. A final layer may be configured as an output layer. Layers between the input layer and output layer are termed hidden layers. An output of the neural network may be calculated as a combination of the network's inputs (where the combination may include applying a non-linear function to the inputs).

A Deep Neural Network is an ANN with at least two hidden layers. A DNN may be a CNN (if it uses convolution filters) i.e. a Deep Convolutional Neural Network (DCNN).

The cognitive state estimation method may include characterising user behaviour in a recording using a CNN or DNN or DCNN. Multiple Neural Networks may be used to create behaviour predictions - for example, in a Generative Adversarial Network including multiple neural networks, competing to produce behaviour predictions.

The method may begin with receipt of a pre-existing recording; the recording may have been recorded prior to receipt in the method. The method may be used to analyse a recording taken of a user who was interviewed by a human and not an avatar. Analysis of the recording to estimate a cognitive state may not be contemporaneous with the recording (and may, for example, take place on a different day).

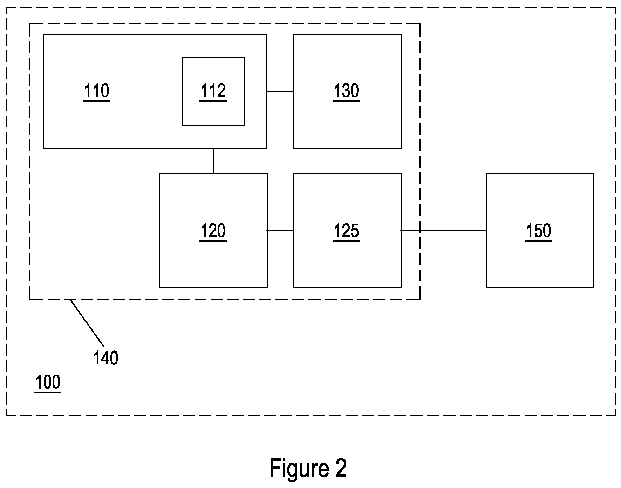

Figure 2 shows a system 100 according to an embodiment. The system 100 comprises: avatar device 110, recording device 120, controller 130, and processor 150.

The avatar device 110 is configured to display/embody the avatar or virtual agent. The avatar device 110 may comprise a display screen 112 configured to display the avatar. For example, the avatar device 110 may be a computer (for example a tablet computer or mobile phone) having a display screen 112. The avatar device 110 may display an avatar (or virtual agent) that resembles a person, on the display screen 112. The avatar device 110 (or part of the avatar device 110) may be shaped like a person, for example - i.e. the avatar device 110 may be a physical robot that resembles a person - providing the avatar. The avatar may appear at least superficially authentic to a human observer.

Tasks set by the avatar device 110 may include reading out loud a text presented to the user, mimicking an expression or sentence shown by the avatar, describing what is shown in a picture, or answering questions asked by the avatar in order to create a 'mood diary'. Although both tasks and questions are referred to here, a question may be a task or a task may include a question.

Tasks and/or questions may be interactive, in that the user's response to an earlier task/question may determine whether a subsequent task/question is assigned, or what a subsequent task/question entails.

Estimates of the user's cognitive state (e.g. about the presence or severity of behaviomedical conditions) may be made based on these active interactions - i.e. based on how the user responds to tasks/questions from the avatar across time steps in the assessment. A human assessor (for example medical professional) may identify a repeated behaviour (for example, facial expression) brought about by a question or task, and make a note of that behaviour to help identify a medical condition. In the present disclosure, a recording of the user is analysed to identify behaviours.

The recording device 120 is configured to obtain a recording of the user from which behaviour primitives may subsequently be extracted. The recording may be a video and/or audio recording. The recording device 120 may be also configured to obtain a recording of the avatar (e.g. as a picture in picture) and/or of the environment of the assessment (for example, the room where the assessment takes place). The avatar device 110 may include the recording device 120.

The recording device 120 may include a video camera - configured to capture video of the user. The recording device 120 may include a microphone to record the user's voice and/or other sounds - for example ambient noise in a room where the assessment takes place, or other voices or interruptions (for example, a fire alarm or phone ringtone). The recording device 120 may be comprised in a user's computing device 140 (such as a laptop, smartphone or tablet, for example). The avatar device 110 may also be comprised in the computing device 140 (which may be more convenient, more practical and more cost-effective than providing the avatar device 110 and recording device 120 as separate devices).

The recording device 120 may be configured to record video and/or audio for the duration of the user being assessed - for example, from the time the user enters the assessment room (for example) until the user exits the assessment room. The recording device 120 may be configured to start recording based on an action of the avatar - for example, the avatar may begin asking a question or reciting a task, and the recording device 120 may be configured to begin recording.

The system 100 may include a controller 130, configured to control the avatar device 110 and/or the recording device 120. In certain embodiments, the controller 130 may be comprised as part of a computing device 140. In other embodiments, the controller 130 may be separate from, and optionally remote from, the avatar device 110 and/or recording device 120.

The controller 130 may control the avatar device 110 to assign a task or ask a question to the user. The controller 130 may control the recording device 120 based on an action of the avatar device 110. The recording device 120 may be configured to begin recording automatically once a question has been asked or a task has been assigned, for example.

An operator may control the avatar device 110 and/or the recording device 120 remotely (e.g. via the Internet and/or via Wi-Fi or another radio frequency protocol).

Use of a remote control may allow an operator to begin, progress and end an assessment of a user from a remote location (for example, outside of an assessment room) - this way, the user is not affected by the presence of another person in the room (for example), resulting in a more controlled assessment. In other embodiments, the assessment of the user may be entirely automatic, available at the time of the user's choosing.

The avatar device 110 may ask the user to begin a recording when they are ready to answer a question or begin a task. The user may be presented with an interface allowing the user to control the recording device 120 or the recording device 120 may be controllable using a voice command. The user may be able to control the recording device 120 using the display screen 112 of the avatar device 110, for example. Control by the user may be restricted - for example, the user may be able to start and stop the recording device 120 but may not be able to delete a recording.

The recording device 120 may be configured to stop recording automatically, for example after a predetermined time period. The predetermined time period may be selected based on the nature of the question/task, for example before the assessment begins. The controller 130 may allow an operator to set the time period and may allow the operator to stop the recording device 120 from recording. The controller 130 may allow the user to stop the recording device 120 from recording - for example via the interface or using a voice command.

Where the recording device 120 is configured to record multiple tasks or responses, the system 100 (for example, the controller 130) may be configured to time stamp the beginning and/or end of each task or response and provide this information as context descriptors. In this way, the length of time taken to perform (i.e. complete or attempt) the task or response is known.

The recording device 120 may include a memory, configured to store images/recordings of the user. The recording device 120 may be configured to send a recording to another device (e.g. a remote server comprising the processor 150) - for example using Wi-Fi or another suitable communication protocol. The recording device 120 may send recordings periodically, for example at the end of an assessment or at the end of a task/response to a question, or may send a recording upon request from an operator. The recording device 120 may be configured to provide a recording to the avatar device 110, which may be configured to process the recording (as described below) and/or transmit the recording to the processor 150.

The system 100 may comprise a context module 125, which obtains context primitives and provides the context primitives to the processor 150. The context module 125 is depicted in Figure 2 as part of the user's computing device 140, but this is not essential. The context module 125 may record aspects of the environment prompting the behaviours from the user, as discussed with reference to Figure 1. The context module 125 may comprise sensors (e.g. light sensors) etc., and may be comprised in the computing device 140. Mobile phones, for example, may comprise suitable sensors for detecting ambient light levels, temperature etc.

The context primitives from the context module 125 and the recording obtained via the recording device 120 are provided to the processor 150, which may be configured to perform all the steps described in Figure 1. Alternatively, the computing device 140 may perform some of the steps. For example, the computing device 140 may perform behaviour primitive extraction on the recording of the user, produce a behaviour map (and/or a context map) and communicate the behaviour map and/or context map) to the processor 150, which may consequently perform the step of estimating the cognitive state (e.g. using a machine learning algorithm). In other embodiments, the recording and context primitives may be provided to the processor 150 (which may be server based, for example in a cloud hosted service), which may perform all the steps of the method described with reference to Figure 1.

The processor 150 may be configured to analyse the recording to identify one or more behaviour primitives as a time series of behaviour descriptors (as already discussed with reference to Figure 1). The time series may represent increments of time throughout the assessment - for example, the duration of the assessment may be 20 minutes and the time series may include 200 units of time, each unit indicating 6 seconds. Figure 3 shows analysis of behaviour primitives across 19 time steps, for example.

The behaviour primitives may comprise numerical values based on how behaviours are exhibited by the user per unit time, for example.

All of the time steps may be of equal length. The system 100 (for example, the processor 150) may be configured to create a time series of equal time step lengths based on a time series where the time steps have differing lengths.

If the user does not exhibit the behaviour primitive during a time step in the recording, the processor may assign the numerical value '0', for example, for that time step.

The behaviour primitives to be extracted may be predetermined - for example, the system 100 (for example, the processor 150) may be programmed to look for one or more specific behaviours in the recording, which may be predetermined. A numerical value per unit time may be assigned based on how the behaviour primitive is exhibited over that unit of time.

In an example, to describe facial muscle actions, the system 100 (or method 10) may describe the intensity of the facial muscle actions as a number between 0 (no activation) and 5 (maximum activation). This is exemplary only - the intensity scale may be chosen based on the nature of the behaviour.

Figure 3 indicates User AU1 and AU12 on a scale between 0 and 5. User AU1 is not exhibited until Time Step 5, for example, represented by a '0'.

The intensity value for the behaviour primitive may be '0' or '1' only, for assessing whether a behaviour primitive is being performed or not (i.e. '1' for Yes, '0' for No). For example, Figure 3 shows User Head nod - '1' may indicate that the user is nodding at that time, and '0' may indicate that the user is not nodding their head.

The same behaviour may have a wider or narrower range of possible intensities, based on how the assessment is conducted. For example, the behaviour may be crying, and the intensities may be '0' or '1' - crying or not crying - or the intensities may be a scale 0 to 10 representing low to high intensity, for example indicating whether the person is not crying, forming tears, or sobbing (or other intensities therebetween), for example. The same behaviour may be analysed across more than one behaviour primitive (as behaviour primitives are elements of expressive behaviour). A behaviour such as crying may be analysed using more than one behaviour primitive - for example, whether the user forms tears and whether the user wipes their eyes and whether the user sniffles, for example.

The user may express themselves verbally during assessment. Longer answers to a question (for example, comprising more than a threshold number of words (e.g. 10, 20 etc.) may indicate a different cognitive state than short answers (for example, single word answers, silence, or inaudible mumbling). A behaviour primitive may comprise a word rate (i.e. how many words per unit time the user is speaking). The combination of context primitives (e.g. what question has just been asked) with behaviours (how many words are subsequently spoken) provides information to the machine learning algorithm that may enable more accurate estimation of the user's cognitive state (than could be determined from the behaviours in isolation of their context).

Use of particular words or phrases may be given a numerical intensity - for example, 'sad', 'stressed', 'happy', or other words (not necessarily emotions) such as 'privacy', 'family', 'work', 'money', for example, may be assigned a numerical value.

In other words, to analyse verbal expression, the method may include assigning numerical values to words. The processor 150 may be configured to process a recording of a user's response to identify words and assign numerical values corresponding with words.

To assign a numerical value to a word, a dictionary lookup may be implemented; every word may be valued based on its place in the dictionary lookup. The system 100 (e.g. the processor 150) may be programmed with the dictionary lookup and may be configured to find the numerical value for a word using the dictionary lookup and to represent the word numerically.

Figure 3 shows the behaviour primitive User Speech - at Time Step '2' the user is speaking, and the numerical representation of the user's speech is '459'. This may represent the 459^{th} word in the dictionary lookup.

At Time Step 17 the user's speech is '0' - zero may be the numerical value assigned to silence or to a response that is inaudible or incomprehensible (i.e. no word or letter can be understood). Based on Figure 3, the user has stopped speaking at Time Step 17 (e.g. having completed an answer).

From the numerical representation of the user's verbal response, it is possible to identify where the user paused (which may be represented by '0' intensity, for example).

Each behaviour primitive may be Fourier transformed. A Fourier transform on a time domain behaviour primitive will produce a frequency domain behaviour primitive, comprising an amplitude and a phase for each of a plurality of frequencies. The number of frequencies may be predetermined, so that the Fourier transform reduces time domain behaviour primitives of arbitrary length to Fourier transformed versions with predetermined length. A behaviour map may be produced by arraying each Fourier transform. Conveniently, a behaviour amplitude map may be formed from the amplitude of each Fourier transform, and a phase behaviour map may be formed from the phase of each Fourier transform.

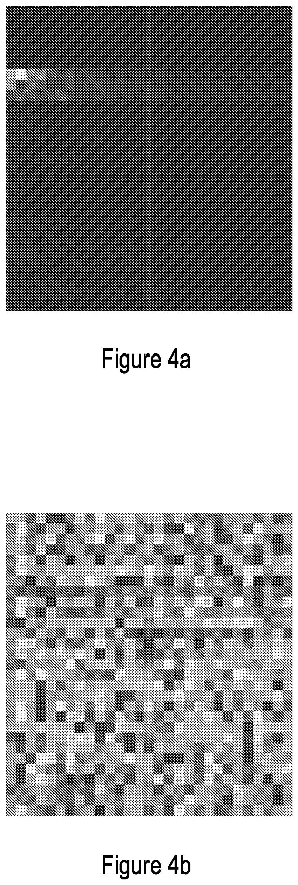

Figure 4a shows an example amplitude behaviour map and Figure 4b shows an example phase behaviour map, both of which have been computed using a Fourier transform.

The amplitude and phase behaviour maps may be combined to produce a single behaviour map (e.g. by vertical concatenation).

The concept of context for behaviomedical estimation can broadly be defined as a collection of environmental parameters, which can be reliably identified during the user's interaction with the avatar. In this way, external parameters - i.e. context - can be considered in the estimation of a cognitive state, which may provide improved accuracy.

Context may be defined as a specific combination of various behaviour primitive elicitation tasks presented to the user combined with avatar properties and the user-avatar interaction telemetric information. Including context in assessment may provide a wider picture of the user's experience during the assessment, and may explain certain responses - for example, a moment of stress may be caused by an interruption, distracting sound or the like. Without context, the moment of stress may appear to be related to the task or question to which the user was responding at the time when a 'stress' behaviour primitive occurred, when with context it may be attributed to an environmental factor (in this example, a disturbance to the assessment).

Context primitives can be presented as time-series context descriptors in the same manner as the behaviour primitives (i.e. turned into a single descriptors per unit of time (e.g. per task)) and might include the following environmental aspects of the user-system interaction:
(i) the system state - for example:
   an avatar's audio-visual properties, such as its facial expression, tone of voice and speech rate; or
   the state of a game such as the score or time taken to achieve a goal; and/or
(ii) details about the settings of user tasks (describe a picture, read a book passage out loud, and the like). This would include various task-dependent parameters such as what picture is presented to the user or what specific part of the book the user is asked to read at any given time. In addition the set of task specific parameters can also be extended with timings, e.g. how long it took for the user to describe a picture and how detailed this description was; and/or
(iii) other environmental parameters not directly related to the user-avatar interaction, which can be reliably identified by the device running the system, such as current time, geographical location, weather conditions (which can be queried online in real-time provided current location). These might also potentially include additional parameters, such as type of background music, if any, ambient noise levels, and more. Various modern mobile device sensors can also be used to determine current illumination levels, movement and position.

**In** addition, context primitives may be identifiers of the user - for example sex, gender, age or other identifiers.

The processor 150 may be configured to determine at least some context primitives from the recording of the user. The processor 150 may, for example, be configured to identify static characteristics or aspects of the person, such as age, gender, etc., from the visual appearance of the person from video or and/or age, gender, etc., from audio in the recording.

The context primitives may be obtained from user identifiers obtained before or after (or during) the assessment, for example the computing device 140 may present the user with a form or ask the user to provide their age (for example - or another user identifier) verbally.

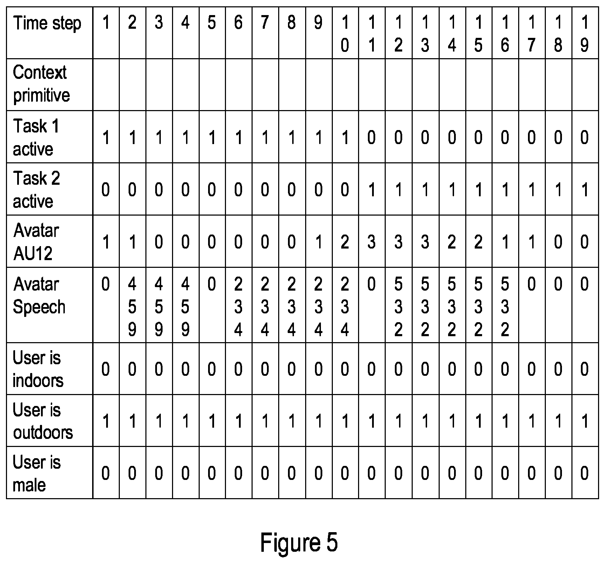

Figure 5 shows an example set of seven context primitives: task 1 active, task 2 active, avatar AU12 (meaning that the avatar is smiling), avatar speech, user is indoors, user is outdoors and user is male. The context primitives in Figure 5 indicate that the user is female ('male' given the value '0') and that the user is outdoors (where outdoors is given the value '1' and indoors is given the value '0').

A context map may be produced in the same way as the behaviour map, by Fourier transforming each of a plurality of time domain context descriptors. The context map may preferably have the same dimensions as the behaviour map.

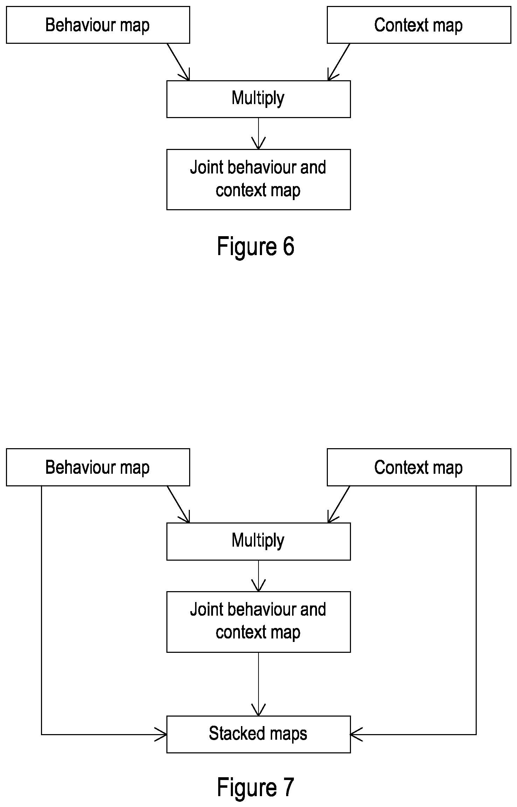

A joint behaviour and context may be formed by combining the behaviour map with the context map, for example by multiplication, as illustrated in Figure 6.

The behaviour map, context map and the joint behaviour may be combined by stacking the respective maps (i.e. the combined FFTs) - as shown in Figure 7. The result is a 6 channel map (i.e. a data cuboid), with a channel for behaviour amplitude, behaviour phase, context amplitude, context phase, joint (behaviour and context) amplitude and joint phase. This approach is similar to the representation of an image using three colour channels (R, G, B) and enables the data to be handled by existing image processing machine learning algorithms (such as CNNs), which may be well optimised for this type of data input.

In order to be able to stack the maps in this way, the behaviour and context maps must have the same number of primitives and the same number of frequencies. If either map has fewer rows (e.g. primitives or descriptors) than the other, it can be padded with additional all-zero lines to make up for the missing data. The processor 150 may be configured to identify that primitives/descriptors are missing and to pad (i.e. fill missing values) of a behaviour map and/or context map with zeros.

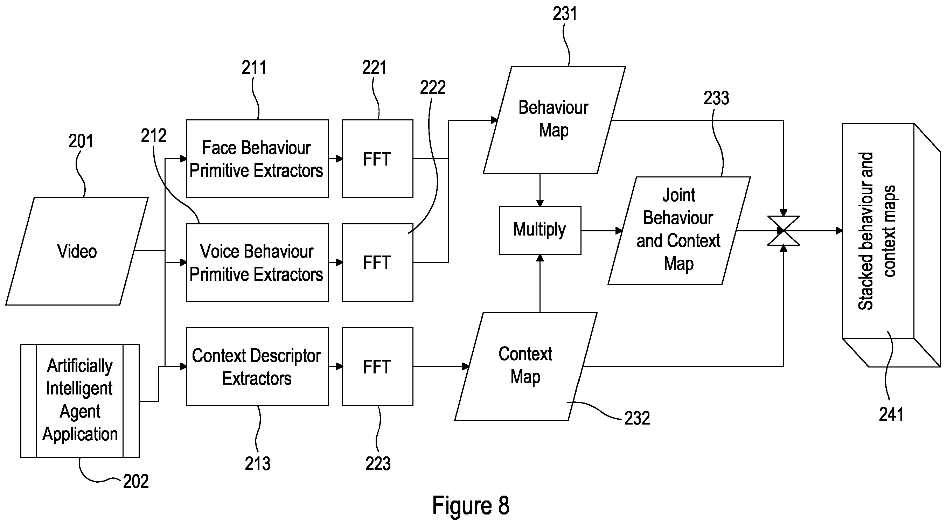

Figure 8 shows a method according to an example embodiment. A video recording 201 of the user is provided to a face behaviour primitive extractor 211, voice behaviour primitive extractor 212 and context primitive extractor 213.

An artificially intelligent agent application 202 (i.e. a virtual agent or avatar) provides data to the context primitive extractor 213 (e.g. a time history of what the avatar was doing during the video, including questions, facial expressions test information etc.).

The face behaviour primitive extractor 211 is configured to extract behaviour primitives relating to the users face, such as Facial Action Coding System Activation Units.

Machine learning algorithms exist in the prior art that are suitable for this task, for example, based on DCNNs.

The voice behaviour primitive extractor 212 is configured to extract behaviour primitives relating to the user's voice, such as the words that are spoken, the speech rate and inflection etc. Again, machine learning algorithms exist in the prior art that are suitable for this task (which may again, be based on DCNNs or any other approach).

Data provided by the agent application 202 and the video 201 are both used by the context primitive extractor 213 to determine the context primitives. For example, as already discussed, the gender of the user may be determined by analysis of the voice and or face of the user, and so on.

Each of the face primitives, voice primitives and context primitives are Fourier transformed 221, 222, 223 (e.g. using a fast Fourier transform or FFT). The Fourier transformed voice and face behaviours are combined (e.g. by concatenation) to produce a behaviour map 231 (as already described). The Fourier transformed context primitives form a context map 232.

The behaviour map 231 and the context map 232 are multiplied to form a joint behaviour and context map 233 (as already described). The maps 231, 232, 233 are then stacked - i.e. forming stacked behaviour and context maps 241 - and may then provide an input to a neural network.

A cognitive state estimate may be made by providing the behaviour, context, and joint map as six input channels to a DNN, CNN or DCNN, for example.

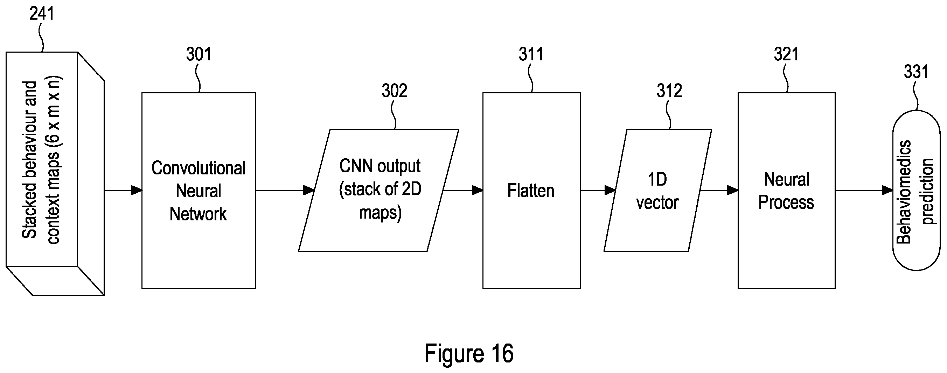

As shown in Figure 16, the stacked behaviour and context maps 241 may be fed to a CNN 301. The CNN 301 may generate an output - the output may be a stack of two-dimensional maps 302.

The applicant has evaluated the benefit of using context features in addition to behaviour features for depression recognition, where the severity of depression in terms of Beck Depression Inventory score was the Behaviomedical estimate. Evaluation was performed on the AVEC 2014 database composed of a number of video recordings of people of various age and gender performing two tasks on camera called Freeform and Northwind. In Freeform subjects are asked various questions about themselves, such as what was their happiest / most prominent childhood memory. These questions may vary from one subject to another. In Northwind subjects are asked to read a book passage. With AVEC-2014 used as a benchmark database it is possible to define two context-related features: task-perming session duration and subject's gender. These two features were appended to the behaviour primitives based feature vector in order to evaluate the effectiveness of context for automatic depression score estimation.

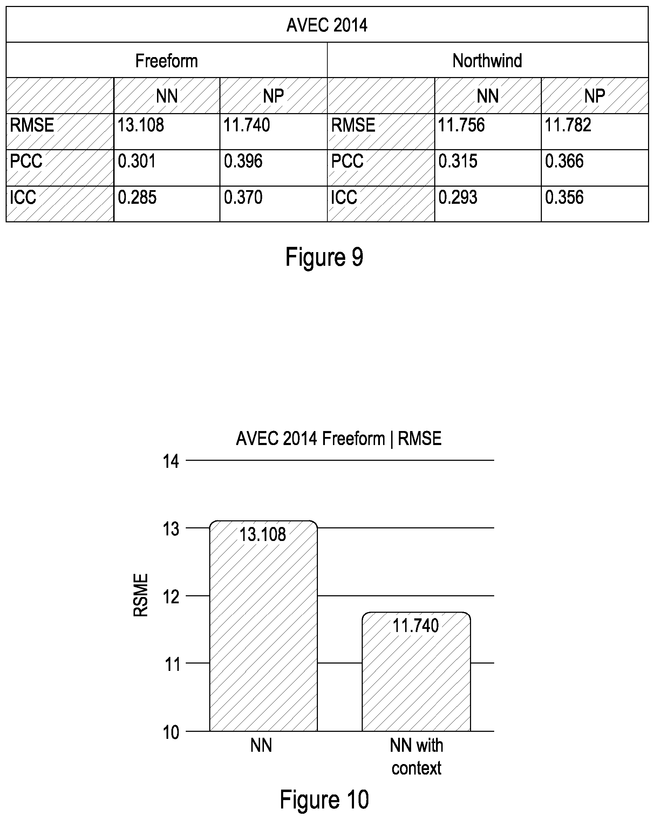

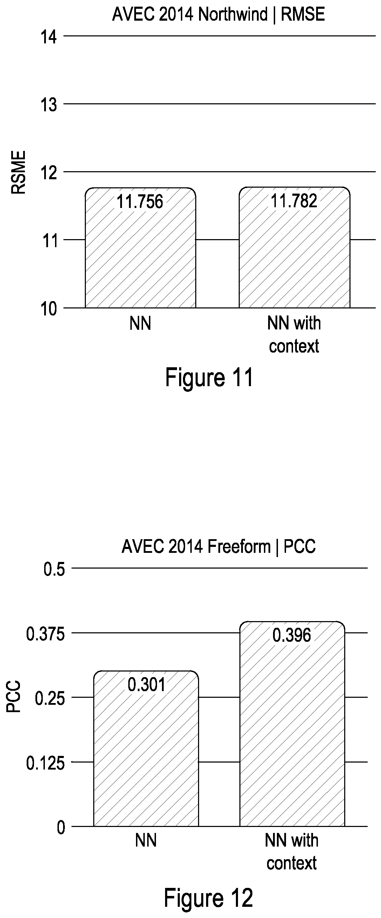

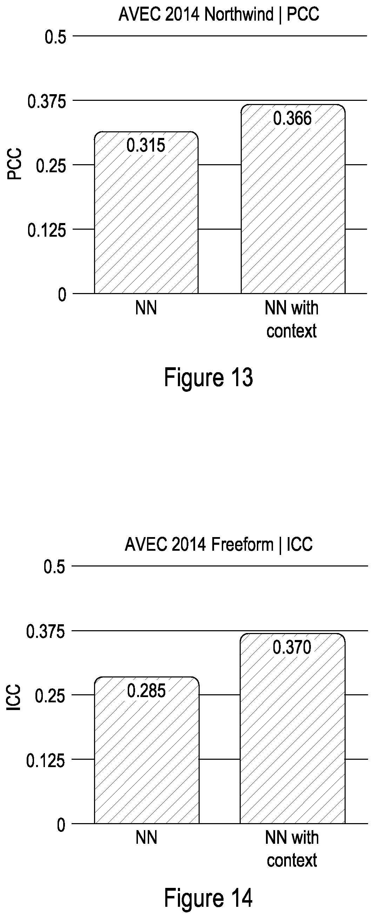

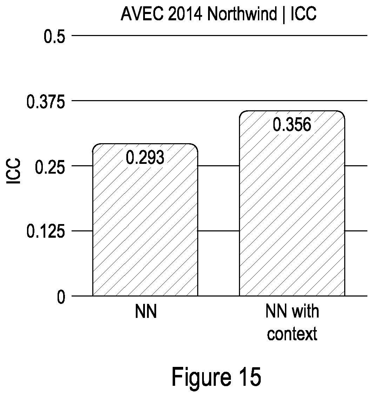

Results of the context features evaluation are provided in Figures 9 to 15. The table provides evaluation results of context features for depression score prediction on the AVEC 2014 database. Figure 10 shows the RMSE error score on AVEC 2014 Freeform task, Figure 11 shows the root mean square error (RMSE) error score on AVEC 2014 Northwind task. Figure 12 shows the Pearson Cross Correlation (PCC) correlation score on AVEC 2014 Freeform task and Figure 13 shows the PCC correlation score on AVEC 2014 Northwind task. Figure 14 shows the Intra-class correlation Coefficient (ICC) correlation score on AVEC 2014 Freeform task and Figure 15 shows the ICC correlation score on AVEC 2014 Northwind task. Evaluation figures clearly indicate the benefit of using context features despite their limited availability in the benchmark dataset. Relative improvements are ~32% and ~16% for the Pearson Cross Correlation (PCC); ~30% and ~22% for the Intra-class correlation Coefficient (ICC) for the Freeform and Northwind tasks respectively; 12% for the root mean square error (RMSE) for the Freeform task; RMSE for the Northwind score is only marginally worse.

The method may be repeated for the same user. The user may have different responses - for example, exhibit different behaviours (which may be similar but not exactly the same) during a different assessment. For example, the user may repeat the assessment in part or in full on the same day (following a break or immediately after an earlier assessment) or at another time. Mixing multiple outcomes to create a new score for the user may overcome errors in individual scores, providing more robust and reliable behaviour predictions.

The method may include using a neural process to generate a behaviomedics prediction.

In certain embodiments, an ANN used for cognitive state estimation may use a layer of a special type of neurons called neural processes (NP) to provide an estimate of cognitive state that is more accurate. In addition, NPs provide a confidence estimation that can be associated with the cognitive state estimate. This confidence estimate may be useful in subsequent clinical diagnosis (e.g. by a clinician or doctor) or when combining multiple behaviomedical estimates. A layer comprising NPs may receive maps (e.g. spectral maps) flattened to a 1D vector directly as input.

In the latter case, an ANN may be configured to receive multiple channels of two-dimensional data as input, such as the stacked behaviour and context maps, as shown in Figure 16. A data format comprising multiple channels of 2D data corresponds with image data, which is an application area in which ANNs have been widely applied. This data format is therefore easy to handle using existing APIs for ANN development.

The CNN 301 outputs 2D maps 302 comprising multiple channels. As discussed above, there may be six channels: i) behaviour primitive frequency amplitudes; ii) behaviour primitive phases; iii) context primitive frequency amplitudes; iv) context primitive phases; v) joint frequency amplitudes; vi) joint phases. The map therefore provides a compact representation of evidence necessary to estimate a cognitive state (e.g. a depression score).. These maps are flattened 311 in a lexicographical manner to a one-dimensional vector 312, which serve as input to the neural process 321. An output of the method of Figure 16 is a behaviomedics prediction 331 - in other words, a cognitive state estimation.

A neural process (NP) combines the best of Gaussian Processes (GP) and Artificial Neural Networks (ANNs). Like GPs, NPs adapt quickly to new observations. Whereas ANNs learn a function that maps an input to a single output, NPs define distributions over functions (like GPs), and thus learn not one function but many functions, thereby capturing the uncertainty in a prediction. Like ANNs, and unlike GPs, NPs are computationally efficient. Like GPs but unlike ANNs, they provide a proper posterior likelihood with their predictions, that is, they output a confidence associated with every prediction.

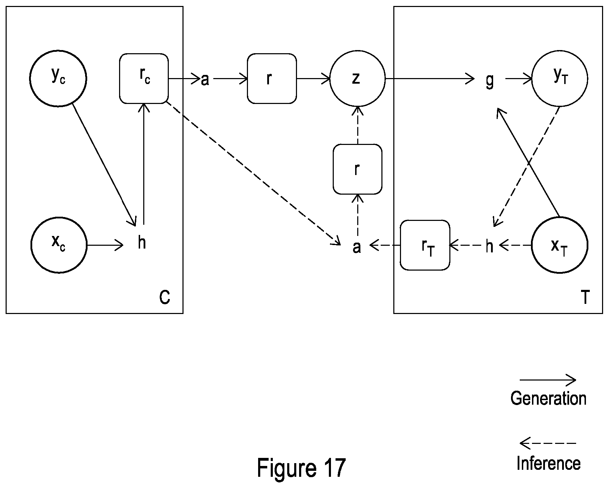

Figure 17 is a diagram of the neural process implementation. Using a neural process may comprise using a global latent variable z and a decoder g to estimate the behaviour from the data. Variables in square boxes correspond to the intermediate representations of NPs and unbound, bold letters correspond to the following computation modules: h - encoder, a - aggregator and g - the decoder. 'h' and 'g' correspond to neural networks and 'a' to the mean function. The continuous lines depict the generative process, the dotted lines the inference.

In the method, the global latent variable may have been determined by encoding conditions by receiving a plurality of condition descriptor pairs. Each condition descriptor pair comprises a true behaviour of a user and data derived from a behaviour map and/or context map for that user, and aggregating the encoded conditions. Descriptor pairs (x_{c}, y_{c}) and (x_{T}, y_{T}) are shown in Figure 17. The bolder circles indicate observed data - x and y represent data, where y is a function of x, C denotes conditions and T denotes 'target' points. The global latent variable z is used with data derived from a behaviour map and/or context map for that user to estimate the user's cognitive state.

The NP model can be boiled down to three core components:
- An encoder h from input space into representation space that takes in pairs of (x, y)i condition values and produces a representation ri = h((x, y)i) for each of the pairs. h is implemented as an artificial neural network;
- An aggregator a that summarises the encoded inputs to an output r, which may be the mean function that takes the average of all ri. Crucially, the aggregator reduces the runtime to O(n + m) where n and m are the number of condition and target points respectively; and
- A conditional decoder g that takes as input the sampled global latent variable z as well as the new target locations xT and outputs the predictions y^T for the corresponding values of f (xT ) = yT.

The method steps of the NP model may be performed by a computing device, for example the processor 150.

The system 100 may include an encoder, an aggregator and a conditional decoder accordingly. The processor 150, for example, may include the encoder, aggregator and conditional decoder.

Each cognitive state estimate made by the system 100 including CNN and NP will have a value and a confidence. Multiple predictions may be combined to improve the confidence, e.g. by asking a user to do multiple different tests, or to repeat the same test multiple times. The cognitive state estimation system 100 may choose not to make a prediction if the confidence is too low, using a threshold determined on a risk-basis.

The present inventor evaluated the benefit of using neural processes rather than standard fully connected layers of neurons for depression recognition, where the severity of depression in terms of Beck Depression Inventory score was the Behaviomedical estimate. Evaluation was performed on the AVEC 2014 database composed of a number of video recordings of people of various age and gender performing two tasks on camera called Freeform and Northwind. In Freeform subjects are asked various questions about themselves, such as what was their happiest / most prominent childhood memory. These questions may vary from one subject to another. In Northwind subjects are asked to read a book passage.

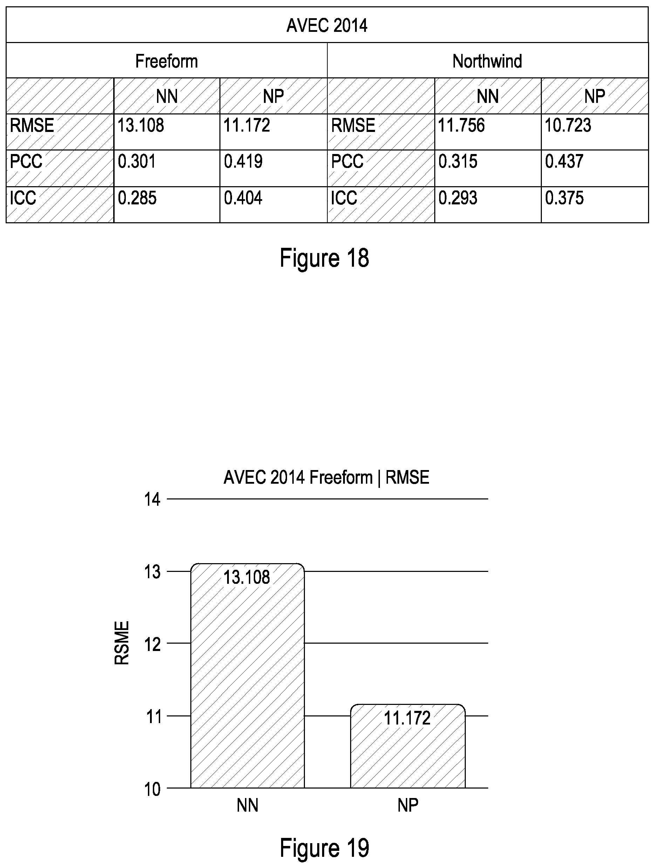

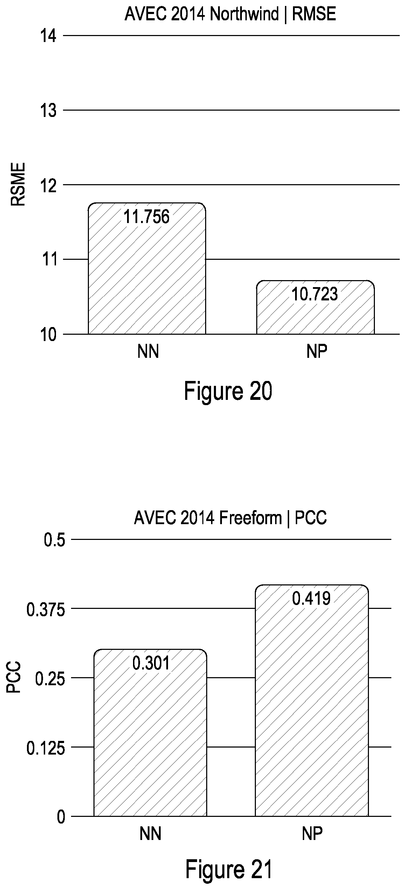

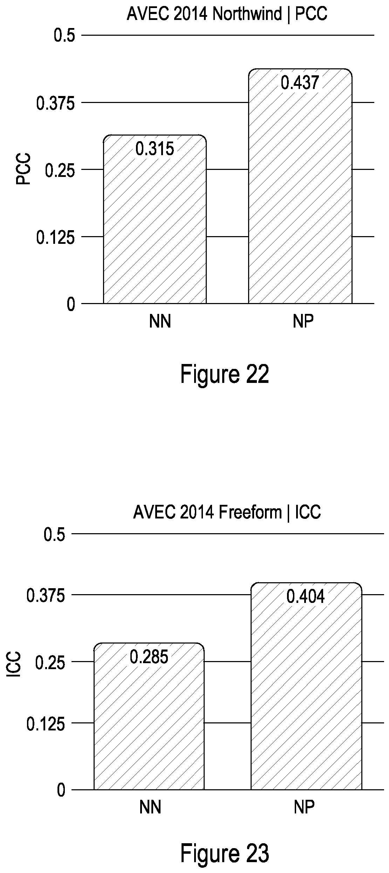

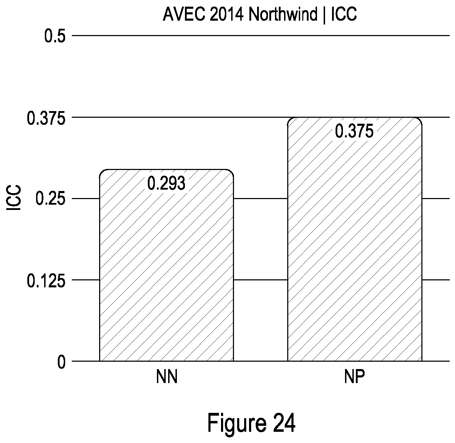

Results of the context features evaluation are provided in Figures 18 to 24. The table provides evaluation results of neural processes against neural networks for depression score prediction on the AVEC 2014 database. Figure 19 shows RMSE error score on AVEC 2014 Freeform task and Figure 20 shows RMSE error score on AVEC 2014 Northwind task. Figure 21 shows PCC correlation score on AVEC 2014 Freeform task and Figure 22 shows PCC correlation score on AVEC 2014 Northwind task. Figure 23 shows ICC correlation score on AVEC 2014 Freeform task and Figure 24 shows ICC correlation score on AVEC 2014 Northwind task.

Evaluation figures clearly indicate the benefit of using neural processes for automatic depression scoring. Relative improvements are ~15% and ~9% for the root mean square error (RMSE) for the Freeform and Northwind tasks respectively; ~39% for the Pearson Cross Correlation (PCC); ~42% and ~28% for the Intra-class correlation Coefficient (ICC) for the Freeform and Northwind tasks respectively.

The method may include delivering feedback to the user during the assessment, or at the end of the assessment, for example. The avatar device 110 may convey a message - for example via the display screen 112 or via a speaker, for example. The virtual agent may be configured to deliver feedback to the user and the mobile computing device 140 may be configured to monitor feedback in real-time.

The contents of the message may be based on results of the cognitive state estimation method. For example, the processor 150 may establish a depression score. The avatar may suggest to the user that a follow-up assessment may be required based on the cognitive state estimation. The user's computing device 140 may allow the user to request a follow-up assessment, book a follow-up assessment, or book an appointment with a medical professional.

The system 100 may include a reporting service component - for example, the avatar device 110 may include a reporting service component - configured to report to a medical professional. The reporting service component may be configured to report results of cognitive state estimation of the user to the user's own doctor or psychiatrist, automatically, for example.

Variations are possible, and the example embodiments are not intended to limit the scope of the invention, which is determined by the accompanying claims.

## Claims

1. A computer implemented cognitive state estimation method, said method comprising:
receiving (11) a recording of a user and at least one context primitive, wherein each context primitive comprises a time series of a single context descriptor per unit of time in numerical form;
extracting (12) at least one behaviour primitive from the recording, wherein each behaviour primitive comprises a time series of a single behaviour descriptor per unit of time in numerical form;
producing (13) a behaviour map (231) from the at least one behaviour primitive, wherein producing the behaviour map (231) comprises performing a Fourier transform on each of the at least one behaviour primitive and assembling the resulting amplitude and phase components into a structured map, wherein the behaviour map comprises a behaviour amplitude map and a behaviour phase map;
producing (14) a context map (232) from the at least one context primitive, wherein producing the context map (232) comprises performing a Fourier transform on each of the at least one context primitive and assembling the resulting amplitude and phase components into a structured map, wherein the context map comprises a context amplitude map and a context phase map; and
estimating (15) a cognitive state of the user using data derived from the behaviour map (231) and the context map (232);
wherein the behaviour map (231) and the context map (232) have the same dimensions;
wherein estimating (15) the cognitive state of the user comprises combining the behaviour map (231) and the context map (232) to create a joint behaviour and context map (233);
wherein combining the behaviour map (231) and the context map (232) comprises multiplying the behaviour map (231) and the context map (232).

2. The method of claim 1, wherein the behaviour map (231) includes multiple behaviour primitives and wherein the context map (232) includes multiple context primitives, the method further comprising determining if the behaviour map (231) or the context map (232) has fewer primitives than the other, and inserting, in the step of producing the behaviour map or the step of producing the context map additional all-zero primitives to the map (231, 232) having fewer primitives where primitives are missing, to create a behaviour map (231) and a context map (232) of equal size.

3. The method of claim 2, wherein the data comprises stacked 2D data comprising a channel for each of the maps (231, 232).

4. The method of any preceding claim, wherein estimating the cognitive state of the user comprises using a convolutional neural network.

5. The method of any preceding claim, wherein estimating (15) a cognitive state of the user comprises using a neural process.

6. The method of claim 5, wherein estimating (15) a cognitive state comprises using a convolutional neural network and the neural process receives an output from the convolutional neural network.

7. The method of any of claims 5 or 6, wherein using a neural process comprises: using a global latent variable and a decoder to estimate the behaviour from the data.

8. The method of claim 7, wherein the global latent variable has been determined by:
encoding conditions by receiving a plurality of condition descriptor pairs, each condition descriptor pair comprising a true behaviour of a user and data derived from a behaviour map (231) and/or context map (232) for that user;
aggregating the encoded conditions.

9. The method of any preceding claim, wherein the recording of the user was obtained by an interaction with a virtual agent.

10. The method of any preceding claim, wherein the recording comprises an audio recording and/or a video recording (201) of the user.

11. The method of any preceding claim, wherein the at least one behaviour primitive comprises a face behaviour primitive.

12. The method of any preceding claim at least one behaviour comprises a voice behaviour primitive.

## Patentansprüche

1. Computerimplementiertes Schätzungsverfahren für einen kognitiven Zustand, wobei das Verfahren Folgendes umfasst:
Empfangen (11) einer Aufzeichnung eines Benutzers und mindestens eines Kontextprimitivums, wobei jedes Kontextprimitivum eine Zeitreihe eines einzelnen Kontextdeskriptors pro Zeiteinheit in numerischer Form umfasst;
Extrahieren (12) mindestens eines Verhaltensprimitivums aus der Aufzeichnung, wobei jedes Verhaltensprimitivum eine Zeitreihe eines einzelnen Verhaltensdeskriptors pro Zeiteinheit in numerischer Form umfasst;
Herstellen (13) einer Verhaltenskarte (231) aus dem mindestens einen Verhaltensprimitivum, wobei das Herstellen der Verhaltenskarte (231) Durchführen einer Fourier-Transformation an jedem des mindestens einen Verhaltensprimitivums und Zusammensetzen der resultierenden Amplituden- und Phasenkomponenten zu einer strukturierten Karte umfasst, wobei die Verhaltenskarte eine Verhaltensamplitudenkarte und eine Verhaltensphasenkarte umfasst;
Herstellen (14) einer Kontextkarte (232) aus dem mindestens einen Kontextprimitivum, wobei das Herstellen der Kontextkarte (232) Durchführen einer Fourier-Transformation an jedem des mindestens einen Kontextprimitivums und Zusammensetzen der resultierenden Amplituden- und Phasenkomponenten zu einer strukturierten Karte umfasst, wobei die Kontextkarte eine Kontextamplitudenkarte und eine Kontextphasenkarte umfasst; und
Schätzen (15) eines kognitiven Zustands des Benutzers unter Verwendung von Daten, die aus der Verhaltenskarte (231) und der Kontextkarte (232) abgeleitet sind;
wobei die Verhaltenskarte (231) und die Kontextkarte (232) die gleichen Abmessungen aufweisen;
wobei das Schätzen (15) des kognitiven Zustands des Benutzers Kombinieren der Verhaltenskarte (231) und der Kontextkarte (232) umfasst, um eine gemeinsame Verhaltens- und Kontextkarte (233) zu erzeugen;
wobei das Kombinieren der Verhaltenskarte (231) und der Kontextkarte (232) Multiplizieren der Verhaltenskarte (231) und der Kontextkarte (232) umfasst.

2. Verfahren nach Anspruch 1, wobei die Verhaltenskarte (231) mehrere Verhaltensprimitiva beinhaltet und wobei die Kontextkarte (232) mehrere Kontextprimitiva beinhaltet, wobei das Verfahren ferner Bestimmen, ob die Verhaltenskarte (231) oder die Kontextkarte (232) weniger Primitiva als die andere aufweist, und Einfügen, in dem Schritt des Herstellens der Verhaltenskarte oder dem Schritt des Herstellens der Kontextkarte, zusätzlicher Primitiva, die alle null sind, zu der Karte (231, 232), die weniger Primitiva aufweist, dort, wo Primitiva fehlen, umfasst, um eine Verhaltenskarte (231) und eine Kontextkarte (232) gleicher Größe zu erzeugen.

3. Verfahren nach Anspruch 2, wobei die Daten gestapelte 2D-Daten umfassen, die einen Kanal für jede der Karten (231, 232) umfassen.

4. Verfahren nach einem der vorhergehenden Ansprüche, wobei das Schätzen des kognitiven Zustands des Benutzers Verwenden eines neuronalen Faltungsnetzwerks umfasst.

5. Verfahren nach einem der vorhergehenden Ansprüche, wobei das Schätzen (15) eines kognitiven Zustands des Benutzers Verwenden eines neuronalen Prozesses umfasst.

6. Verfahren nach Anspruch 5, wobei das Schätzen (15) eines kognitiven Zustands Verwenden eines neuronalen Faltungsnetzwerks umfasst und der neuronale Prozess eine Ausgabe von dem neuronalen Faltungsnetzwerk empfängt.

7. Verfahren nach einem der Ansprüche 5 oder 6, wobei das Verwenden eines neuronalen Prozesses Folgendes umfasst: Verwenden einer globalen latenten Variable und eines Decodierers, um das Verhalten anhand der Daten zu schätzen.

8. Verfahren nach Anspruch 7, wobei die globale latente Variable durch Folgendes bestimmt wurde:
Codieren von Bedingungen durch Empfangen einer Vielzahl von Bedingungsdeskriptorpaaren, wobei jedes Bedingungsdeskriptorpaar ein wahres Verhalten eines Benutzers und Daten, die aus einer Verhaltenskarte (231) und/oder Kontextkarte (232) für diesen Benutzer abgeleitet wurden, umfasst;
Aggregieren der codierten Bedingungen.

9. Verfahren nach einem der vorhergehenden Ansprüche, wobei die Aufzeichnung des Benutzers durch eine Interaktion mit einem virtuellen Agenten erlangt wurde.

10. Verfahren nach einem der vorhergehenden Ansprüche, wobei die Aufzeichnung eine Audioaufzeichnung und/oder eine Videoaufzeichnung (201) des Benutzers umfasst.

11. Verfahren nach einem der vorhergehenden Ansprüche, wobei das mindestens eine Verhaltensprimitivum ein Gesichtsverhaltensprimitivum umfasst.

12. Verfahren nach einem der vorhergehenden Ansprüche, mindestens ein Verhalten ein Sprachverhaltensprimitivum umfasst.

## Revendications

1. Procédé d'estimation d'état cognitif mis en œuvre par ordinateur, ledit procédé comprenant :
la réception (11) d'un enregistrement d'un utilisateur et d'au moins une primitive de contexte, dans lequel chaque primitive de contexte comprend une série temporelle d'un unique descripteur de contexte par unité de temps sous forme numérique ;
l'extraction (12) d'au moins une primitive de comportement à partir de l'enregistrement, dans lequel chaque primitive de comportement comprend une série temporelle d'un unique descripteur de comportement par unité de temps sous forme numérique ;
la production (13) d'une carte de comportement (231) à partir de l'au moins une primitive de comportement, dans lequel la production de la carte de comportement (231) comprend la réalisation d'une transformée de Fourier sur chacune de l'au moins une primitive de comportement et l'assemblage des composants d'amplitude et de phase résultants en une carte structurée, dans lequel la carte de comportement comprend une carte d'amplitude de comportement et une carte de phase de comportement ;
la production (14) d'une carte de contexte (232) à partir de l'au moins une primitive de contexte, dans lequel la production de la carte de contexte (232) comprend la réalisation d'une transformée de Fourier sur chacune de l'au moins une primitive de contexte et l'assemblage des composants d'amplitude et de phase résultants en une carte structurée, dans lequel la carte de contexte comprend une carte d'amplitude de contexte et une carte de phase de contexte ; et
l'estimation (15) d'un état cognitif de l'utilisateur en utilisant des données dérivées provenant de la carte de comportement (231) et de la carte de contexte (232) ;
dans lequel la carte de comportement (231) et la carte de contexte (232) ont les mêmes dimensions ;
dans lequel l'estimation (15) de l'état cognitif de l'utilisateur comprend la combinaison de la carte de comportement (231) et de la carte de contexte (232) pour créer une carte conjointe de comportement et de contexte (233) ;
dans lequel la combinaison de la carte de comportement (231) et de la carte de contexte (232) comprend la multiplication de la carte de comportement (231) et de la carte de contexte (232).

2. Procédé selon la revendication 1, dans lequel la carte de comportement (231) inclut de multiples primitives de comportement et dans lequel la carte de contexte (232) inclut de multiples primitives de contexte, le procédé comprenant en outre la détermination du fait que la carte de comportement (231) ou la carte de contexte (232) a moins de primitives que l'autre, et l'insertion, dans l'étape de production de la carte de comportement ou l'étape de production de la carte de contexte, de primitives supplémentaires entièrement nulles dans la carte (231, 232) ayant moins de primitives là où des primitives sont manquantes, pour créer une carte de comportement (231) et une carte de contexte (232) de taille égale.

3. Procédé selon la revendication 2, dans lequel les données comprennent des données 2D empilées comprenant un canal pour chacune des cartes (231, 232).

4. Procédé selon une quelconque revendication précédente, dans lequel l'estimation de l'état cognitif de l'utilisateur comprend l'utilisation d'un réseau de neurones convolutif.

5. Procédé selon une quelconque revendication précédente, dans lequel l'estimation (15) d'un état cognitif de l'utilisateur comprend l'utilisation d'un processus neuronal.

6. Procédé selon la revendication 5, dans lequel l'estimation (15) d'un état cognitif comprend l'utilisation d'un réseau de neurones convolutif et le processus neuronal reçoit une sortie provenant du réseau de neurones convolutif.

7. Procédé selon l'une quelconque des revendications 5 ou 6, dans lequel l'utilisation d'un processus neuronal comprend : l'utilisation d'une variable latente globale et d'un décodeur pour estimer le comportement à partir des données.

8. Procédé selon la revendication 7, dans lequel la variable latente globale a été déterminée par :
le codage de conditions par la réception d'une pluralité de paires de descripteurs de condition, chaque paire de descripteurs de condition comprenant un comportement réel d'un utilisateur et des données dérivées provenant d'une carte de comportement (231) et/ou d'une carte de contexte (232) pour cet utilisateur ;
l'agrégation des conditions codées.

9. Procédé selon une quelconque revendication précédente, dans lequel l'enregistrement de l'utilisateur a été obtenu par une interaction avec un agent virtuel.

10. Procédé selon une quelconque revendication précédente, dans lequel l'enregistrement comprend un enregistrement audio et/ou un enregistrement vidéo (201) de l'utilisateur.

11. Procédé selon une quelconque revendication précédente, dans lequel l'au moins une primitive de comportement comprend une primitive de comportement de visage.

12. Procédé selon une quelconque revendication précédente, dans lequel l'au moins un comportement comprend une primitive de comportement de voix.
